# EUROPÄISCHE PATENTSCHRIFT

(11) **EP 2 081 019 B1**
(45) Veröffentlichungstag und Bekanntmachung des Hinweises auf die Patenterteilung: **19.10.2011**
(21) Anmeldenummer: 08105866.1
(22) Anmeldetag: 26.11.2008
(51) Int. Cl.: G01N 27/41, G01N 27/407

(54) **Gassensor und Verfahren zur Detektion von NOx-Teilchen in einem Gasstrom**
Gas sensor and method for detecting NOx-particles in a gas flow
Capteur de gaz et procédé de détection de petites pièces de NOx dans un flux de gaz

(30) Priorität: 15.01.2008 DE 102008004372
(43) Veröffentlichungstag der Anmeldung: 22.07.2009
(73) Patentinhaber: ROBERT BOSCH GMBH, 70442 Stuttgart (DE)
(72) Erfinder: Schneider, Jens, 71229 Leonberg (DE); Heimann, Detlef, 70839 Gerlingen (DE); Reinhardt, Goetz, 71032 Boeblingen (DE); Runge, Henrico, 70435 Stuttgart (DE); Diehl, Lothar, 70839 Gerlingen (DE); Ruth, Juergen, 70469 Stuttgart (DE); Seiler, Thomas, 70195 Stuttgart (DE)

(56) Entgegenhaltungen:
- WO-A1-02/090967
- DE-A1- 10 048 240
- US-A1- 2005 029 127

## Beschreibung

Die Erfindung betrifft einen Gassensor, sowie ein Verfahren mit deren Hilfe Teilchen in einem Gasstrom, insbesondere in NOₓ in einem Abgasstrom eines Kraftfahrzeugs, detektiert werden können.

### Stand der Technik

Aus DE 10 2005 056 522 A1 ist ein nach dem Doppelkammerprinzip arbeitender Gassensor bekannt, der zwei elektrochemische Pumpzellen aufweist, die jeweils eine in einem Messraum angeordnete Elektrode aufweisen, mit deren Hilfe gasförmige Teilchen zwischen dem Messraum und der Umgebung gepumpt werden können. Die erste Pumpzelle ist mit dem Gasstrom verbunden, um die im Gasstrom zu detektierenden Teilchen zu detektieren. Die zweite Pumpzelle ist als Referenz mit einem Referenz-Gasstrom verbunden, der im Wesentlichen keine zu detektierende Teilchen aufweist. Die Elektroden der Pumpzellen wirken jeweils mit einer ihnen zugeordneten gegenpoligen Gegenelektrode zusammen, sodass es möglich ist an den jeweiligen Elektroden ein Messsignal, insbesondere einen elektrischen Strom, abzugreifen. Das Verhältnis des von der ersten Elektrode erhaltenen Messsignals mit dem von einer zweiten Elektrode erhaltenen Referenz-Messsignals ist ein Maß für die Konzentration der zu detektierenden Teilchen, insbesondere NOₓ.

Nachteilig bei einem derartigen Gassensor ist, dass insbesondere sehr geringe Konzentrationen nicht detektiert werden können, da derartige Messsignale im Signalrauschen untergehen. Insbesondere können derartige Messsignale nicht von möglichen Störungen, beispielsweise durch Offsetströme, unterschieden werden.

Es ist die Aufgabe der Erfindung einen Gassensor sowie ein Verfahren zur Detektion von Teilchen in einem Gasstrom zu schaffen, die jeweils auch geringe Konzentrationen valide messen können.

### Offenbarung der Erfindung

Die Lösung der Aufgabe erfolgt erfindungsgemäß durch einen Gassensor mit den Merkmalen des Anspruchs 1 sowie ein Verfahren zur Detektion von Teilchen in einem Gasstrom mit den Merkmalen des Anspruchs 3. Vorteilhafte Ausgestaltungen der Erfindung sind in den Unteransprüchen angegeben.

Der erfindungsgemäße Gassensor zur Detektion von NOₓ in einem Gasstrom weist eine elektrochemische Pumpzelle zum Pumpen von Teilchen auf. Die Pumpzelle weist einen Messraum auf zwischen dem und dem Gasstrom die Teilchen gepumpt werden können. In dem Messraum ist eine erste Elektrode angeordnet, mit deren Hilfe die zu detektierenden NOₓ-Teilchen zumindest indirekt über ein Pumpen von O-Ionen, gepumpt werden. In demselben Messraum ist ferner eine zweite Elektrode angeordnet, mit deren Hilfe O₂-Teilchen gepumpt werden können. Die erste Elektrode und die zweite Elektrode sind mit einer Steuereinrichtung verbindbar, die derart eingerichtet ist, dass in einem ersten Zeitraum nur die erste Elektrode und in einem zweiten Zeitraum nur die zweite Elektrode betrieben wird. Insbesondere ist es möglich, dass sich mehrere erste Zeiträume und mehrere zweite Zeiträume alternierend abwechseln.

Durch den alternierenden Betrieb der Elektroden, bei dem entweder nur die erste Elektrode oder nur die zweite Elektrode betrieben wird, findet auch nur alternierend eine elektrochemische Reaktion an der jeweiligen Elektrodenoberfläche statt. Dadurch ist es möglich, dass sich diejenigen Teilchen, die in einem Zeitraum nicht detektiert werden, innerhalb dieses Zeitraums aufkonzentrieren können. Diese erhöhte Konzentration der Teilchen kann in dem jeweils anderen Zeitraum besser detektiert werden, da ein höherer Messwert von der jeweiligen Elektrode erhältlich ist. Dieser Effekt wird dadurch verstärkt, dass sowohl die erste Elektrode als auch die zweite Elektrode in einem gemeinsamen Messraum angeordnet sind. Dadurch erhöht sich die Konzentration der gerade nicht detektierten Teilchen nicht nur durch eine Diffusion dieser Teilchen aus dem Gasstrom in den Messraum, sondern auch dadurch, dass die jeweils anderen Teilchen bzw. deren Ionen eine elektrochemische Reaktion erfahren können. Da sich durch die zeitverzögerte elektrochemische Reaktion der zu detektierenden Teilchen bzw. deren Ionen die Konzentration des jeweils anderen Teilchens erhöht, ist es möglich, auch bei geringen Konzentrationen der zu detektierenden Teilchen eine hierzu proportionale Konzentrationserhöhung herbeizuführen, die valide gemessen werden kann. Insbesondere kann beispielsweise die erste Elektrode sowohl O₂-Teilchen als auch NOₓ-Teilchen detektieren, so dass in einem derartigen Fall das Messsignal der zweiten Elektrode intern von dem Messsignal der ersten Elektrode abgezogen werden kann, um ein für NOₓ-Teilchen proportionales Messergebnis zu erhalten.

Erfindungsgemäß ist in dem Messraum ein Absorbermedium zum Absorbieren der zu detektierenden NOₓ Teilchen angeordnet. Das Absorbermedium ist insbesondere in Abhängigkeit von der Absorptionsfähigkeit für die zu detektierenden Teilchen ausgewählt. Das Absorbermedium weist vorzugsweise BaCO₃ und/oder Al₂O₃ auf, um besonders gut NOₓ-Teilchen absorbieren zu können. Durch die Absorption der zu detektierenden Teilchen, kann die Konzentration im Messraum zusätzlich beeinflusst werden. Erfindungsgemäß ist eine Heizvorrichtung zum Heizen des Absorbermediums vorgesehen, die derart eingerichtet ist, dass in dem Absorbermedium eine erste Temperatur T₁ und eine von der Ersttemperatur T₁ verschiedene zweite Temperatur T₂ einstellbar ist, wobei das Absorbermedium bei der ersten Temperatur T₁ die zu detektierenden Teilchen absorbiert und bei der zweiten Temperatur T₂ die absorbierten zu detektierenden Teilchen desorbiert. Bei der ersten Temperatur T₁ kann somit in dem Messraum der Pumpzelle eine besonders geringe Konzentration der zu detektierenden Teilchen eingestellt werden, während bei der zweiten Temperatur T₂ eine besonders hohe Konzentration der zu detektierenden Teilchen erreicht werden kann. Da bei der zweiten Temperatur T₂ eine über der Zeit aufkumulierte Menge der zu detektierenden Teilchen abgegeben wird, ist die Integration des während der zweiten Temperatur T₂ gemessenen Messwerte für die zu detektierenden Teilchen über der Zeit ein Maß für die Konzentration der zu detektierenden Teilchen im Gasstrom. Aufgrund der Aufkumulierung der zu detektierenden Teilchen sind auch besondere geringe Konzentrationen besonders valide, das heißt, ohne wesentliche Messfehler, detektierbar.

Besonders bevorzugt sind die erste Elektrode und die zweite Elektrode im Wesentlichen gleich geformt. Beide Elektroden können also die gleiche Größe, die gleiche Dicke und die gleiche Geometrie aufweisen. Durch eine im Wesentlichen identische geometrische Gestaltung der Elektroden ergeben sich bei beiden Elektroden im Wesentlichen die gleichen Offsetströme, so dass sich bei einem Vergleich der Messwerte der ersten Elektrode mit den Messwerten der zweiten Elektrode systemimmanente Messfehler ausgleichen. Dadurch kann insbesondere ein Messwerteverhältnis, beispielsweise das Verhältnis der Konzentrationen von NOₓ zu O₂ besonders valide bestimmt werden.

Die Erfindung betrifft ferner ein Verfahren zu Detektion von Teilchen NOₓ in einem Gasstrom, bei dem ein Gassensor einem Gasstrom, insbesondere einem Abgasstrom eines Kraftfahrzeugs, ausgesetzt wird. Der Gassensor kann insbesondere, wie vorstehend beschrieben, aus- und weitergebildet sein. Der Gassensor weist eine Pumpzelle auf, die eine erste Elektrode zum Pumpen der zu detektierenden Teilchen, insbesondere NOₓ-Teilchen, und eine zweite Elektrode zum Pumpen von O₂-Teilchen aufweist, wobei die erste Elektrode insbesondere sowohl NOₓ-Teilchen als auch O₂-Teilchen pumpen kann. Das Pumpen von NOₓ-Teilchen erfolgt zumindest indirekt über ein Pumpen von O-Ionen. Erfindungsgemäß wird in einem ersten Zeitraum nur die erste Elektrode betrieben und in einem zweiten Zeitraum nur die zweite Elektrode. Insbesondere erfolgt der erste Zeitraum und der zweite Zeitraum mehrfach alternierend hintereinander.

Wie bereits vorstehend anhand des Gassensors erläutert, kann sich während des ersten Zeitraums die zu detektierenden Teilchen, die mit der zweiten Elektrode detektiert werden sollen, aufkonzentrieren und umgekehrt. Die aufkonzentrierte Menge der jeweiligen zu detektierenden Teilchen kann dann mit weniger Messfehlern und höherer Genauigkeit, das heißt valider, detektiert werden.

In einer bevorzugten Ausführungsform wird zunächst nur die zweite Elektrode betrieben, bis ein im Wesentlichen konstanter Messwert von der zweiten Elektrode erhalten wird. Durch diesen Betrieb wird innerhalb der Pumpzelle ein im Wesentlichen stationärer O₂-Massenstrom erreicht, bei dem eine minimale O₂-Konzentration vorliegt. In der Pumpzelle kann dann eine besonders hohe Konzentration von NOₓ-Teilchen vorliegen. Die minimale Konzentration an O₂-Teilchen in dem Messraum der Pumpzelle kann an dem konstanten Messwert für die zweite Elektrode erkannt werden, da der konstante Messwert anzeigt, dass genauso viele O₂-Teilchen elektrochemisch umgesetzt werden wie O₂-Teilchen in den Messraum der Pumpzelle gelangen.

Erfindungsgemäß weist die Pumpzelle ein Absorbermedium zur Absorption der detektierenden Teilchen auf. In einem ersten Betriebsmodus kann das Absorbermedium mit einer ersten Temperatur T₁ betrieben werden, bei der das Absorbermedium die zu detektierenden Teilchen absorbiert. Nachfolgend kann am zweiten Betriebsmodus das Absorbermedium mit einer zweiten Temperatur T₂ betrieben werden, bei der das Absorbermedium die zuvor absorbierten zu detektierenden Teilchen desorbiert. Um insbesondere NOₓ-Teilchen zu absorbieren, kann das Absorbermedium insbesondere BaCO₃ aufweisen. Durch die zwischenzeitliche Absorption der zu detektierenden Teilchen kann nach einer Desorption eine deutlich höhere Konzentration detektiert werden, die valider gemessen werden kann. Insbesondere werden während des zweiten Betriebsmodus, insbesondere mehrfach alternierend, nur die erste Elektrode in einem ersten Zeitraum und nur die zweite Elektrode in einem zweiten Zeitraum betrieben. Der alternierende Betrieb der beiden Elektroden erfolgt somit insbesondere zu einer Zeit, in der die zuvor absorbierten zu detektierenden Teilchen desorbiert werden. Besonders bevorzugt wird das Betreiben nur der ersten Elektrode in dem ersten Zeitraum und das Betreiben nur der zweiten Elektrode in dem zweiten Zeitraum beendet, wenn der von der ersten Elektrode erhältliche Messwert im Wesentlichen konstant ist. Der konstante Messwert der ersten Elektrode zeigt an, dass keine weiteren zu detektierenden Teilchen mehr von dem Absorbermedium desorbiert werden.

Vorzugsweise werden die im zweiten Zeitraum gemessenen Messwerte aufintegriert und werden als Maß für die Konzentration der zu detektierenden Teilchen im Gasstrom verwendet. Wenn die ersten Zeiträume und die zweiten Zeiträume hinreichend klein gewählt werden, lässt sich insbesondere die zuvor aufkumulierte Menge absorbierter zu detektierender Teilchen besonders genau messen. Wenn der erste Zeitraum und der zweite Zeitraum im Wesentlichen gleich groß sind, entspricht das Integral der gemessenen Messwerte der ersten Elektrode über der Zeit im Wesentlichen der halben Menge der über einen bestimmten Zeitraum absorbierten Teilchen.

### Zeichnungen

Nachfolgend wird die Erfindung unter Bezugnahme auf die anliegenden Zeichnungen anhand eines bevorzugten Ausführungsbeispiels näher erläutert.

Es zeigen:
- Fig. 1:: eine schematische Ansicht des erfindungsgemäßen Gassensors und
- Fig. 2:: ein schematisches Diagramm qualitativer Messwerte bei Anwendung des erfindungsgemäßen Verfahrens.

### Beschreibung der Ausführungsbeispiele

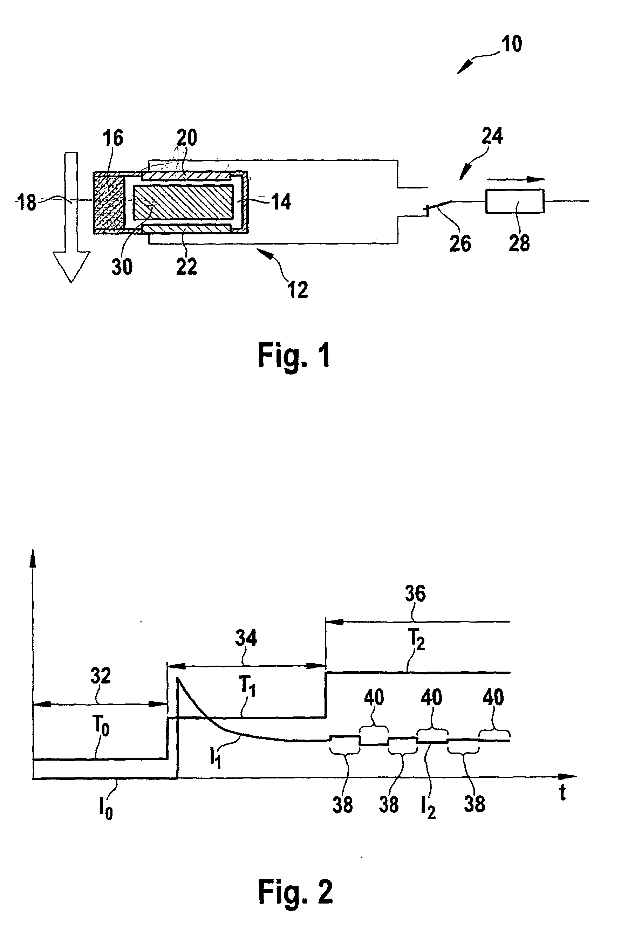

Der in Fig. 1 dargestellte Gassensor 10 weist eine Pumpzelle 12 auf, die einen Messraum 14 aufweist. Der Messraum 14 ist über eine Diffusionsbarriere 16 mit einem Gasstrom 18 verbunden, damit nicht zu viele Teilchen gepumpt werden. In dem Messraum 14 der Pumpzelle 12 ist eine erste Elektrode 20 angeordnet, die platindotiert oder insbesondere rhodiumdotiert sein kann, um NOₓ zu pumpen. In demselben Messraum 14 derselben Pumpzelle 12 ist eine zweite Elektrode 22 angeordnet, die insbesondere golddotiert ist, um O₂ zu pumpen. Die erste Elektrode 20 und die zweite Elektrode 22 sind mit einer Steuereinrichtung 24 verbunden, die über einen Schalter 26 alternierend nur die erste Elektrode 20 oder nur die zweite Elektrode 22 mit einer elektrischen Spannung beaufschlagen kann. Über eine mit dem Schalter 26 verbundene Messeinrichtung 28 kann ein Messwert, insbesondere ein elektrischer Strom, gemessen werden.

Der Messraum 14 der Pumpzelle 12 weist ferner ein Absorbermedium 30 auf, das beispielsweise im Wesentlichen aus BaCO₃ besteht. Aus dem Gasstrom 18, der insbesondere der Abgasstrom eines Kraftfahrzeugs ist, kann über die Diffusionsbarriere 16 NOₓ in den Messraum 14 hinein diffundieren und von dem Absorbermedium 30 absorbiert werden. Zu einem späteren Zeitpunkt kann das Absorbermedium 30 so stark erwärmt werden, dass die zuvor absorbierten NOₓ-Teilchen desorbiert werden und von der ersten Elektrode 20 detektiert werden können.

Wie in Fig. 2 dargestellt, kann der in Fig. 1 dargestellte Gassensor 10 insbesondere derart betrieben werden, dass zunächst ein Sammelzeitraum 32 vorgesehen ist, in dem bei einer Sammeltemperatur T₀ NOₓ-Teilchen von dem Absorbermedium 30 absorbiert werden. Während des Sammelzeitraums 32 kann sowohl die erste Elektrode 20 als auch die zweite Elektrode 22 abgeschaltet sein, so dass ein Messwert I₀ gemessen wird, der 0 beträgt. Anschließend wird der Gassensor 10, beziehungsweise die Pumpzelle 12 auf eine Betriebstemperatur von ca. 600°C aufgeheizt. Es ergibt sich eine erste Temperatur T₁ bei der das Absorbermedium 30 immer noch NOₓ-Teilchen absorbieren kann. In diesem ersten Betriebsmodus 34 ist die Temperatur hoch genug, dass sich eine ausreichende Diffusion durch die Diffusionsbarriere 16 hindurch ergibt. Das in dem Messraum 14 eindiffundierte O₂ wird von der zweiten Elektrode 22 elektrochemisch umgesetzt, so dass sich für den gemessenen Strom im ersten Betriebsmodus 34 ein Messwertverlauf I₁ ergibt.

Sobald der Messwertverlauf I₁ auf ein im Wesentlichen konstanten Wert abgefallen ist und sich somit ein stationärer Zustand für die O₂-Teilchen eingestellt hat, wird in einem zweiten Betriebsmodus 36 gewechselt, bei dem eine zweite Temperatur T₂ eingestellt wird, bei der die zuvor absorbierten NOₓ-Teilchen aus dem Absorbermedium 30 desorbieren und sich der Messwertverlauf I₂ ergibt. Gleichzeitig wird alternierend nur der Strom der ersten Elektrode 20 oder nur der Strom der zweiten Elektrode 22 gemessen, so dass sich alternierend mehrere erste Zeiträume 38 ergeben, in denen nur die erste Elektrode 20 gemessen wird und mehrere zweite Zeiträume 40, in denen nur die zweite Elektrode 22 gemessen wird. Der über der Zeit integrierte Verlauf der ersten Zeiträume 38 ist ein Maß für die Menge an NOₓ, das aus dem Absorbermedium 30 desorbiert wurde und somit ein Maß für die NOₓ -Konzentration des Gasstroms 18. Sobald während des ersten Zeitraums 38 und dem zweiten Zeitraum 40 derselbe Messwert erhalten wird, ist im Wesentlichen sämtliches NOₓ aus dem Absorbermedium 30 desorbiert, so dass dieses Verfahren erneut begonnen werden kann.

Für eine Initialisierung der Messwerte der beiden Elektroden 20, 22 können beispielsweise Messwerte aufgenommen werden, wenn sich im Gasstrom 18 nur Umgebungsluft befindet. Dies ist bei Kraftfahrzeug insbesondere im Schubbetrieb der Fall.

## Patentansprüche

1. Gassensor zur Detektion von NOₓ-Teilchen, in einem Gasstrom (18), mit einer elektrochemischen Pumpzelle (12), die einen Messraum (14) aufweist, zum Pumpen von NOₓ-Teilchen zwischen dem Messraum (14) und dem Gasstrom (18), einer in dem Messraum (14) angeordneten ersten Elektrode (20) der Pumpzelle (12), die platindotiert oder rhodiumdotiert ist zum Pumpen von NOₓ-Teilchen, und einer in dem selben Messraum (14) angeordneten zweiten Elektrode (22) derselben Pumpzelle (12), die golddotiert ist zum Pumpen von O₂-Teilchen, wobei die erste Elektrode (20) und die zweite Elektrode (22) mit einer Steuereinrichtung (24) verbunden sind, die derart eingerichtet ist, dass in einem ersten Zeitraum (38) nur die erste Elektrode (20) und in einem zweiten Zeitraum (40) nur die zweite Elektrode (22) betrieben wird, wobei in dem Messraum (14) ein Absorbermedium (30) zum Absorbieren der zu detektierenden NOₓ-Teilchen angeordnet ist, wobei eine Heizvorrichtung zum Heizen des Absorbermediums (30) vorgesehen ist, die derart eingerichtet ist, dass in dem Absorbermedium (30) eine erste Temperatur T₁ und eine von der ersten Temperatur T₁ verschiedene zweite Temperatur T₂ eingestellt ist, wobei das Absorbermedium (30) bei der ersten Temperatur T₁ die zu detektierenden NOₓ-Teilchen absorbiert und bei der zweiten Temperatur T₂ die absorbierten zu detektierenden NOₓ-Teilchen desorbiert.

2. Gassensor nach einem der Ansprüche 1 bis 3 **dadurch gekennzeichnet, dass** die erste Elektrode (20) und die zweite Elektrode (22) im Wesentlichen gleich geformt sind.

3. Verfahren zur Detektion von NOₓ-Teilchen, in einem Gasstrom (18), mit den Schritten:
Bereitstellen eines dem Gasstrom (18) ausgesetzten Gassensors (10) nach einem der Ansprüche 1 oder 2,
Betreiben nur der ersten Elektrode (20) in einem ersten Zeitraum (38) und
Betreiben nur der zweiten Elektrode (22) in einem zweiten Zeitraum (40).

4. Verfahren nach Anspruch 3, bei dem zunächst nur die zweite Elektrode (22) betrieben wird bis ein im Wesentlichen konstanter Messwert von der zweiten Elektrode (22) erhalten wird.

5. Verfahren nach Anspruch 3 oder 4, bei dem die Pumpzelle (12) ein Absorbermedium (30) zur Absorption der zu detektierenden Teilchen aufweist und bei dem in einem ersten Betriebsmodus (34) das Absorbermedium (30) mit einer ersten Temperatur T₁, bei der das Absorbermedium die zu detektierenden Teilchen absorbiert, und nachfolgend in einem zweiten Betriebsmodus (36) das Absorbermedium (30) mit einer zweiten Temperatur T₂, bei der das Absorbermedium (30) die zuvor absorbierten zu detektierenden Teilchen desorbiert, betrieben wird.

6. Verfahren nach Anspruch 5, bei dem während des zweiten Betriebsmodus (36), insbesondere mehrfach alternierend, nur die erste Elektrode (20) in dem ersten Zeitraum (38) und nur die zweite Elektrode (22) in dem zweiten Zeitraum (40) betrieben wird.

7. Verfahren nach Anspruch 5 oder 6, bei dem das Betreiben nur der ersten Elektrode (20) in dem ersten Zeitraum (38) und das Betreiben nur der zweiten Elektrode (22) in dem zweiten Zeitraum (40) beendet wird, wenn der von der ersten Elektrode (20) erhältliche Messwert im Wesentlichen konstant ist.

8. Verfahren nach einem der Ansprüche 3 bis 7, bei dem die im ersten Zeitraum (38) gemessenen Messwerte aufintegriert werden und als Maß für die Konzentration der zu detektierenden NOₓ-Teilchen im Gasstrom (18) verwendet werden.

## Claims

1. Gas sensor for the detection of NOₓ particles in a gas flow (18), having an electrochemical pumping cell (12), which has a measurement chamber (14), for pumping NOₓ particles between the measurement chamber (14) and the gas flow (18), having a first electrode (20) of the pumping cell (12), which first electrode is arranged in the measurement chamber (14) and is platinum-doped or rhodium-doped for the pumping of NOₓ particles, and having a second electrode (22) of the same pumping cell (12), which second electrode is arranged in the same measurement chamber (14) and is gold-doped for the pumping of O₂ particles, wherein the first electrode (20) and the second electrode (22) are connected to a control device (24) which is set up such that only the first electrode (20) is operated in a first time period (38) and only the second electrode (22) is operated in a second time period (40), wherein an absorber medium (30) for absorbing the NOₓ particles to be detected is arranged in the measurement chamber (14), wherein a heating device for heating the absorber medium (30) is provided, which heating device is set up such that a first temperature T₁ and a second temperature T₂ which differs from the first temperature T₁ is set in the absorber medium (30), wherein the NOₓ particles to be detected are absorbed by the absorber medium (30) at the first temperature T₁, and the absorbed NOₓ particles to be detected are desorbed by the absorber medium (30) at the second temperature T₂.

2. Gas sensor according to Claim 1, **characterized in that** the first electrode (20) and the second electrode (22) are of substantially the same form.

3. Method for the detection of NOₓ particles in a gas flow (18), having the steps:
providing a gas sensor (10) according to either of Claims 1 and 2, which gas sensor is exposed to the gas flow (18),
operating only the first electrode (20) in a first time period (38), and
operating only the second electrode (22) in a second time period (40).

4. Method according to Claim 3, in which firstly only the second electrode (22) is operated until a substantially constant measurement value is obtained from the second electrode (22).

5. Method according to Claim 3 or 4, in which the pumping cell (12) has an absorber medium (30) for the absorption of the particles to be detected, and in which, in a first operating mode (34), the absorber medium (30) is operated with a first temperature T₁ at which the absorber medium absorbs the particles to be detected, and subsequently in a second operating mode (36), the absorber medium (30) is operated with a second temperature T₂ at which the absorber medium (30) desorbs the previously absorbed particles to be detected.

6. Method according to Claim 5, in which, during the second operating mode (36), in particular in a repeatedly alternating fashion, only the first electrode (20) is operated in the first time period (38) and only the second electrode (22) is operated in the second time period (40).

7. Method according to Claim 5 or 6, in which the operation of only the first electrode (20) in the first time period (38) and the operation of only the second electrode (22) in the second time period (40) is ended when the measurement value obtained from the first electrode (20) is substantially constant.

8. Method according to one of Claims 3 to 7, in which the measurement values measured in the first time period (38) are integrated and used as a measure for the concentration of the NOₓ particles to be detected in the gas flow (18).

## Revendications

1. Capteur de gaz pour la détection de particules de NOₓ dans un flux de gaz (18), avec une cellule de pompage électrochimique (12), qui présente une chambre de mesure (14), pour le pompage de particules de NOₓ entre la chambre de mesure (14) et le flux de gaz (18), une première électrode (20) de la cellule de pompage (12) disposée dans la chambre de mesure (14), qui est dopée au platine ou dopée au rhodium pour le pompage de particules de NOₓ, et une deuxième électrode (22) de la même cellule de pompage (12) disposée dans la même chambre de mesure (14), qui est dopée à l'or pour le pompage de particules de O₂, dans lequel la première électrode (20) et la deuxième électrode (22) sont reliées à un dispositif de commande (24), qui est conçu de telle manière que seule la première électrode (20) fonctionne pendant un premier laps de temps (38) et que seule la deuxième électrode (22) fonctionne pendant un deuxième laps de temps (40), dans lequel un milieu absorbant (30) pour l'absorption des particules de NOₓ à détecter est disposé dans la chambre de mesure (14), dans lequel il est prévu un dispositif de chauffage pour le chauffage du milieu absorbant (30), qui est conçu de telle manière qu'une première température T₁ et une deuxième température T₂ différente de la première température T₁ soient réglées dans le milieu absorbant (30), dans lequel le milieu absorbant (30) absorbe à la première température T₁ les particules de NOₓ à détecter et désorbe à la deuxième température T₂ les particules de NOₓ à détecter absorbées.

2. Capteur de gaz selon la revendication 1, **caractérisé en ce que** la première électrode (20) et la deuxième électrode (22) ont sensiblement la même forme.

3. Procédé pour la détection de particules de NOₓ dans un flux de gaz (18), présentant les étapes suivantes:
préparer un capteur de gaz (10) selon l'une quelconque des revendications 1 ou 2, exposé au flux de gaz (18),
activer uniquement la première électrode (20) pendant un premier laps de temps (38), et
activer uniquement la deuxième électrode (22) pendant un deuxième laps de temps (40).

4. Procédé selon la revendication 3, dans lequel on active d'abord uniquement la deuxième électrode (22) jusqu'à ce que l'on obtienne une valeur de mesure sensiblement constante à la deuxième électrode (22).

5. Procédé selon la revendication 3 ou 4, dans lequel la cellule de pompage (12) contient un milieu absorbant (30) pour l'absorption des particules à détecter et dans lequel, dans un premier mode de fonctionnement (34), on active le milieu absorbant (30) avec une première température T₁ à laquelle le milieu absorbant absorbe les particules à détecter, et ensuite, dans un deuxième mode de fonctionnement (36), on active le milieu absorbant (30) avec une deuxième température T₂ à laquelle le milieu absorbant (30) désorbe les particules à détecter antérieurement absorbées.

6. Procédé selon la revendication 5, dans lequel, pendant le deuxième mode de fonctionnement (36), en particulier plusieurs fois en alternance, on active uniquement la première électrode (20) dans le premier laps de temps (38) et uniquement la deuxième électrode (22) dans le deuxième laps de temps (40).

7. Procédé selon la revendication 5 ou 6, dans lequel on termine l'activation uniquement de la première électrode (20) dans le premier laps de temps (38) et l'activation uniquement de la deuxième électrode (22) dans le deuxième laps de temps (40), lorsque la valeur de mesure disponible à la première électrode (20) est sensiblement constante.

8. Procédé selon l'une quelconque des revendications 3 à 7, dans lequel on intègre les valeurs de mesure mesurées dans le premier laps de temps (38) et on les utilise comme mesure de la concentration des particules de NOₓ à détecter dans le flux de gaz (18).
